# EUROPEAN PATENT SPECIFICATION

(11) **EP 2 572 593 B1**
(45) Date of publication and mention of the grant of the patent: **22.06.2016**
(21) Application number: 12188676.6
(22) Date of filing: 10.12.2007
(51) Int. Cl.: A23P 10/00, A23C 9/20, A23L 33/00, A61K 35/20

(54) **Compositions of human lipids and methods of making and using same**
Zusammensetzungen menschlicher Fette sowie Verfahren zu ihrer Herstellung und Verwendung
Compositions de lipides humains et procédés de fabrication et leur utilisation

(30) Priority: 08.12.2006 US 869150 P
(43) Date of publication of application: 27.03.2013
(62) Divisional of application: 07865463.9
(73) Proprietor: Prolacta Bioscience, Inc., City of Industry, CA 91746 (US)
(72) Inventor: Medo, Elena M., Murrieta, CA California 92563 (US); Eaker, Scott, Monrovia, CA 91016 (US)
(74) Representative: Walton, Seán Malcolm

(56) References cited:
- US-A- 4 762 822
- JENSEN R G ET AL: "Lipids in human milk and infant formulas", ANNUAL REVIEW OF NUTRITION, ANNUAL REVIEWS INC., PALO ALTO, CA, US, vol. 12, 1 July 1992 (1992-07-01), pages 417-441, XP009112668, ISSN: 0199-9885

## Description

### TECHNICAL FIELD

The present disclosure relates to compositions that include lipids from human milk and methods of making and using such compositions.

### BACKGROUND

Nutritional support can be administered to the patients in need of it, e.g., enterally or parenterally (e.g., by a process called total parenteral nutrition). Both enteral and parenteral formulas generally include carbohydrates, lipids, proteins, fiber, and/or vitamins and minerals, depending on the needs of a patient. Parenteral formulas may include other additives, such as heparin, H2 blocker etc. The sources of lipids used in parenteral formulas are generally bovine milk, soy, safflower oil, olive oil, and fish oil.

### SUMMARY

This disclosure features compositions that include lipids from human milk, methods of obtaining such compositions, and methods of using such compositions to provide nutrition to patients, e.g., human patients.

Nutritional support can be administered to the patients in need of it, e.g., enterally or parenterally (e.g., by a process called total parenteral nutrition). Both enteral and parenteral formulas generally include carbohydrates, lipids, proteins, fiber, and/or vitamins and minerals, depending on the needs of a patient. Parenteral formulas may include other additives, such as heparin, H2 blockers etc. The sources of lipids used in parenteral formulas are generally bovine milk, soy, safflower oil, olive oil, and fish oil. The present disclosure features compositions that include lipids from human milk and that can be used to provide nutritional support to human patients. The compositions can include omega-3 and/or omega-6 fatty acids (and their derivatives and precursors). The compositions may be supplemented, if desired, with, e.g., emulsifiers, preservatives and/or other nutritional constituents. Without being bound by theory, it is believed that use of human lipids may reduce the incidence of liver damage that can occur in patients undergoing TPN that includes non-human lipid sources. Use of human lipids in enteral compositions may also be more beneficial when compared to non-human lipids.

The methods featured herein can be carried out with large volumes of the starting material, e.g., human milk, or pooled human milk. The volumes can be in the range of about 75 liters to about 2,000 liters/lot of the starting material.

In one aspect, the invention is as defined in claim 1.

Embodiments include one or more of the following features.

In one embodiment, the obtaining step includes obtaining from about 75 liters to about 2,000 liters of whole human milk.

In another embodiment, processing the cream portion in step (c) includes precipitating protein components out of the cream portion and/or separating the precipitated protein from the cream portion. Processing the cream portion includes purifying the cream portion, e.g., diafiltrating the cream portion. The processing can further include ultrafiltration. The method further includes, after step (c), adding one or more constituents selected from the group consisting of: an emulsifier, a preservative (e.g., an antioxidant, e.g., alpha-tocopherol, vitamin C, a carotenoid and a retinoid), a diluent, and an osmolality modifier(e.g., glycerin). The method further includes after step (c), adding one or more constituents selected from the group consisting of: a vitamin, a mineral, and a carbohydrate.

In one embodiment, the method further provides for parenterally administering the composition obtained after step (d) to a subject, e.g., a human subject, e.g., a human infant, e.g., a premature infant. The subject can have a nutritional disease or disorder. The administration is parenteral administration, e.g., part of total parenteral nutritional administration.

The disclosure also provides for treating a subject having a nutritional disease or disorder. The method includes administering to the subject the compositions featured herein (e.g., the composition that includes human lipid fraction from human milk and an emulsifier), thereby treating the subject. The subject is a human subject, e.g., a human infant, e.g., a premature infant.

The terms "premature," "preterm," and "low-birth-weight (LBW)" infants are used interchangeably and refer to infants born less than 37 weeks gestational age and/or with birth weights less than 2500 gm.

By "whole milk" is meant human milk from which no fat has been removed.

As used herein, the term "critically-ill patients" refers to patients who are suffering from a total or partial dysfunction of the gastro-intestinal tract due to prematurity, disease or stress of injury such as surgery, cancer, acute diabetes, AIDS, malnutrition, trauma, ulcerative colitis, necrotizing enterocolitis, or sepsis. The term "critically-ill patients," as used herein, is also intended to include hypercatabolic patients. These critically-ill individuals are often hospitalized and must be administered most or all of their daily nutritional requirements enterally or parenterally in order to sustain protein synthesis and to minimize the likelihood of becoming malnourished, to maintain nutritional status, or to improve nutritional status.

Unless defined otherwise, technical and scientific terms used herein have the same meaning as that commonly understood by one of skill in the art.

### BRIEF DESCRIPTION OF THE DRAWINGS

FIG. 1 is a flow chart of an embodiment of making a composition that includes human lipids for TPN.
FIG. 2 is a flow chart of an embodiment of making a composition that includes human lipids for enteral administration.

### DETAILED DESCRIPTION

This disclosure features compositions that include lipids from human milk, methods of obtaining such compositions and methods of using such compositions to provide nutrition to patients, e.g., human patients.

Human subjects, e.g., critically-ill patients, post-operative patients, patients suffering from a nutritional disease or disorder, and/or premature infants, often require administration of nutritional support. Nutritional support can be administered to patients enterally or parenterally (e.g., by a process called total parenteral nutrition or TPN).

Total parenteral nutrition (TPN) is a practice of intravenous feeding, which bypasses eating and digestion. Enteral nutrition is a practice of tube feeding, e.g., nasogastric, transpyloric, and percutaneous. Both enteral and parenteral formulas generally include carbohydrates, lipids, proteins, fiber, and/or vitamins and minerals, depending on the needs of a patient.

As mentioned above, lipids are an important component of both the enteral and the total parenteral nutrition. The present disclosure provides methods of obtaining compositions that include human lipids and that can be used in both enteral and parenteral nutrition, e.g., premature infant enteral and parenteral nutrition. The compositions can include omega-3 and/or omega-6 fatty acids (and their derivatives and precursors). The compositions can be supplemented with other constituents, e.g., emulsifiers, preservatives, diluents, osmolality modifiers, and/or other nutritional components (e.g., vitamins, minerals, and/or carbohydrates). Without being bound by theory, it is believed that use of human lipids may reduce the incidence of liver damage that can occur in patients undergoing TPN that includes non-human lipid sources. Use of human lipids in enteral compositions may also be more beneficial when compared to use of non-human lipids.

The methods featured herein can be carried out with large volumes of the starting material, e.g., human milk, or pooled human milk. The volumes can be in the range of about 75 liters to 2,000 liters/lot of starting material.

### Enteral and Parenteral Nutrition

Total parenteral nutrition (TPN) is a practice of intravenous feeding, which bypasses eating and digestion. Enteral nutrition is a practice of tube feeding, e.g., nasogastric, transpyloric, and percutaneous. Each practice has known benefits and drawbacks, and skilled practitioners can choose one mode over the other, depending on individual patient's needs. Both enteral and parenteral formulas generally include carbohydrates, lipids, proteins, fiber, and/or vitamins and minerals, depending on the needs of a patient. Parenteral formulas may include other additives, such as heparin,
H2 blocker etc.

Subjects in need of nutritional support include, e.g., premature infants, post-surgical patients, patients with a nutritional disease or disorder, and critically-ill patients. For example, premature infants may have limited capacity of the stomach, deformed organs, and/or immaturity of metabolism. Post-surgical patients may not have the ability to feed themselves. Critically-ill patients may need nutritional support for a variety of reasons. Critically-ill patients can include those with severe burns, trauma, and catabolic states.

One group of patients who need supplemental nutrition includes those with malnutrition. For example, protein calorie malnutrition is a common complicating condition in patients with alcoholic chronic liver disease (Mendenhall et al., Am. J. Med. 76:211-222, 1984; Mendenhall et al., Am. J. Clin. Nutr. 43:213-218, 1986) and nonalcoholic chronic liver disease (O'Keefe et al. Lancet 2:615-617, 1980; Morgan et al. Gut 1976;17:113-118). Patients with end stage liver disease complicated by portal hypertension are particularly likely to be malnourished and, when hospitalized, frequently require active nutritional therapy. While the effects of malnutrition in chronic liver disease on fatty acid nutrition have not been extensively studied, it is believed that because of an increased resting energy expenditure, fat malabsorption and abnormal fat catabolism, these patients may have significant abnormalities in fatty acid metabolism (Cabre et al., Am. J. Gastroent. 83:712-717, 1988; Palombo et al., Gastroent. 93: 1170-1177, 1987). Without being bound by theory, one potential mechanism for such a disturbance would be an inadequate intake of essential fatty acids as part of the global protein calorie malnutrition.

A patient who is administered a TPN solution containing only saccharides, electrolytes and amino acids for a prolonged period generally will exhibit the symptoms of an essential fatty acid deficiency, such as scale efflorescence, eczematoid eruption, retarded wound healing, thrombocytopenia, fat swelling, anemia and the like. The deficiency is developed within about four to six weeks from the start of TPN, especially when the energy source is only saccharides. Such essential fatty acid deficiency (which is rare in humans who obtain routine nutrition) can be avoided by administering an essential fatty acid-rich fat emulsion concomitantly during TPN therapy.

Lipid emulsions or other preparations containing, e.g., omega-3 fatty acids, are used in TPN and enteral nutritional treatments. For example, they are useful in treating severe trauma victims and subjects with disseminate intravascular coagulation.

### Lipids Derived from Human Milk

As noted above, the sources of lipids used in TPN generally include bovine milk, soy, olive oil, and fish oil. This disclosure provides methods of obtaining and using lipids from human milk. The compositions featured herein contain human lipids, which can include both omega-3 and omega-6 fatty acids. Without being bound by theory, it is believed that use of human lipids in TPN may reduce the incidence of liver damage that can occur in patients undergoing TPN that includes non-human lipid sources. Similarly, use of human lipids may be beneficial in enteral nutrition.

Human milk comprises about 100,000 different molecular entities - proteins, lipids, carbohydrates, vitamins, and trace minerals. Human milk contains about 3% to 5% of total lipids that exist as emulsified globules coated with a membrane derived from a secreting cell (Jensen et al., J. Dairy Sci. 73:223-240, 1990). The lipids present in human milk include: triacylglycerol (about 98%), phospholipids (about 0.5-1%), and sterols (about 0.2 to 0.5%, e.g., cholesterol) (Jensen *et al., supra*). Triacylglycerols serve as, e.g., storage molecules of fatty acids.

Different fatty acids in lipids have different physiological, biochemical and, in some cases, pharmacological properties. Dietary fatty acids are classified, e.g., according to their chain length. Long chain fatty acids contain 16 carbons or more and can be further characterized by the number of double bonds contained in their structure (into saturated, monounsaturated and polyunsaturated subgroups). The two fatty acids essential in human nutrition are linoleic acid and alpha-linolenic acid, from which polyunsaturated fatty acids (PUFA) of the omega-6 series and omega-3 series, respectively, are formed. Examples of omega-3 fatty acids include eicosapentaenoic acid (EPA) and docosahexonenoic acid (DHA). Examples of omega-6 fatty acids include gamma linoleic acid (GLA), dihomogamma linoleic acid (DGLA), and arachidonic acid (AA). The body cannot convert omega-3 fatty acids to omega-6 fatty acids or *vice versa.*

Lipids in human milk represent the main source of energy for the breastfed baby and provide essential nutrients, such as fat-soluble vitamins and PUFA. Long chain polyunsaturated fatty acids (LC-PUFA) are key structural components of cellular membranes and are deposited in the growing brain and the retina during perinatal development.
Addition of preformed LC-PUFA to human milk lipids has been shown to be related to improved visual acuity and development of cognitive functions during the first year of life in the recipient infants.

Human milk content of fatty acids can vary, depending on the diet of the mother. For example, docosahexaenoic acid (DHA) is a 20 carbon omega-3 fatty acid. If the mother often eats fish high in DHA , her milk will generally contain higher DHA levels than the milk of a mother with less access to fish. Consequently, human milk may require DHA supplementation to insure sufficient amounts of DHA. DHA supplementation is typically accompanied by arachidonic acid supplementation. For example, U.S. Pat. No. 5,492,938, describes a method of obtaining DHA from dinoflagellates and its use in pharmaceutical composition and dietary supplements.

The methods described herein include generating compositions with specific amounts of desired beneficial omega-3 and omega-6 fatty acids.

### Human Milk Donors

The starting material of the methods featured herein is human milk. Human milk is collected from donors by systematic methods, described, e.g., in U.S. Application Serial No. 11/947,580 filed November 29, 2007, and in U.S. Patent Application No. 11/526,127 (U.S. 2007/0098863). The methods featured herein can be carried out with large volumes of human milk, e.g., pooled human milk. The volumes can be in the range of about 75 liters to about 2,000 liters/lot of starting material. Once the milk is collected, it can be frozen, pooled, and processed to obtain a composition of human lipids.

### Methods of Obtaining Human Lipid Compositions for TPN

Previous methods of obtaining human lipid compositions have been described in U.S. Pat. No. 4762822. The methods described herein generate human lipid compositions suitable, e.g., for TPN administration. The methods will utilize some components generated in the methods of obtaining human milk fortifiers (HMFs) described in U.S. Application Serial No. 11/947,580 filed on November 29, 2007, as further explained below.

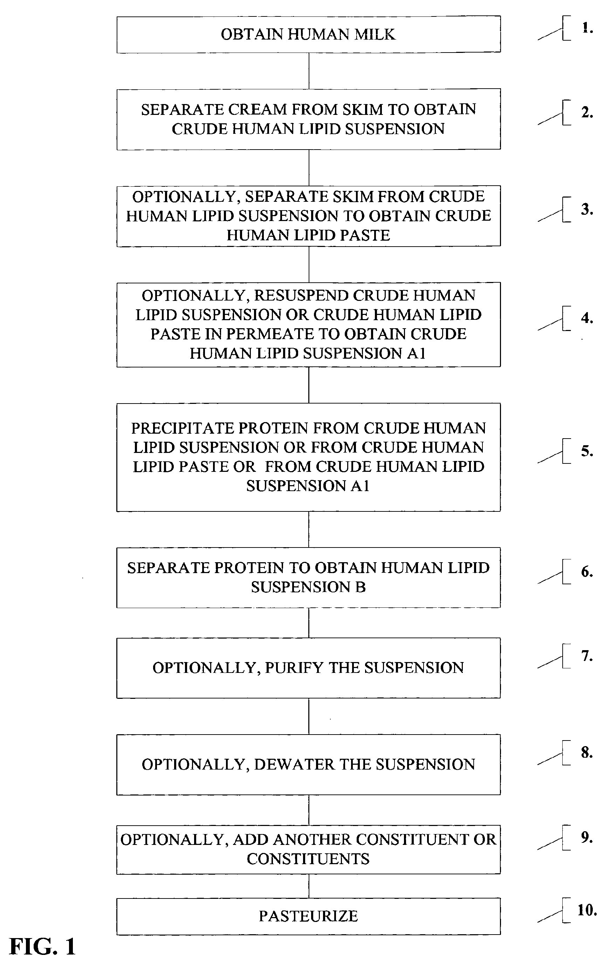

Referring to Fig. 1, step 1, human milk is obtained from donors, as described above. If frozen, the milk can be thawed, pooled, warmed to about 25°C, and genetically screened for contaminants, e.g., viral contaminants, as described in U.S.S.N. 11/947,580. The milk then undergoes filtering, e.g., through about a 200 micron filter, and heat treatment (at about 63°C or greater for about 30 minutes or more). The milk is transferred to a separator, e.g., a centrifuge, to separate the cream from the skim (step 2). This process intermediate is referred to as Crude Human Lipid Suspension A. The skim can be transferred into a second processing tank, e.g., until a filtration step.

In an optional step 3, the cream component can be separated once more (e.g., by centrifugation) to remove additional skim. This process intermediate is referred to as Crude Human Lipid Paste.

In an optional step 4, the Crude Human Lipid Paste can be re-suspended in excess permeate from the HMF manufacturing process of U.S.S.N. 11/947,580. This process intermediate is referred to as Crude Human Lipid Suspension A1. In the HMF process, following separation of cream and skim the skim undergoes further filtration, e.g., ultrafiltration. Ultrafiltration is a type of membrane filtration, in which hydrostatic pressure forces a liquid against a semipermeable membrane; solids and high-molecular weight solutes are retained, while water and low-molecular weight solutes pass through the membrane. Here, this process concentrates the nutrients in the skim milk by filtering out the water. The water obtained during the concentration is referred to as the permeate. This permeate can be used in optional step 4 of the present methods.

In step 5, any remaining protein components not bound to lipids can be precipitated from solution by manipulating various parameters (e.g., temperature, ionic strength, and solvent (e.g., ethanol or polyethylene glycol (PEG) concentration). Such precipitation techniques are known by those skilled in the art. The precipitated proteins can be used in various nutritional supplements.

In step 6, precipitated proteins can be separated from the lipid suspension (e.g., by centrifugation). This process intermediate is referred to as Human Lipid Suspension B.

In an optional step 7, soluble and insoluble salts, other ions, and small molecular entities can be removed from the lipid suspension via a purification process (e.g., diafiltration). Diafiltration is a process, in which ultrafiltration membranes are used to remove or lower the concentration of salts or solvents, or to replace buffer salts from solutions that contain large molecules, such as lipids. This process intermediate is referred to as Diafiltrate A. In optional step 8, the diafiltrate can be dewatered (e.g., by ultrafiltration). This process intermediate is referred to as Purified Human Lipid Suspension.

In an optional step 9, additional constituent(s) can be added to the resulting composition. The constituents can include: an emulsifier, a preservative, a diluent, an osmolality modifier, and a nutritional component (e.g., mineral, vitamin, and carbohydrate). Examples of such constituents are discussed below.

In step 10, the Purified Human Lipid Suspension will be pasteurized, yielding a final product. Pasteurization methods are known in the art. For example, the suspension can be pasteurized at a minimum of about 66 degrees Celsius with the air space maintained at about 69 degrees Celsius for a minimum of about thirty minutes. In one embodiment, the pasteurization can be a short-time (less than about 10 minutes) and ultra-high temperature pasteurization.

Specific order and/or combination of these steps can be adjusted, if desired.

### Methods of Obtaining Human Lipid Compositions for Enteral Administration

The methods described herein generate human lipid compositions suitable, e.g., for enteral administration. The methods will utilize some components generated in the methods of obtaining human milk fortifiers (HMFs) described in U.S. Application Serial No. 11/947,580 filed on November 29, 2007, as further explained below.

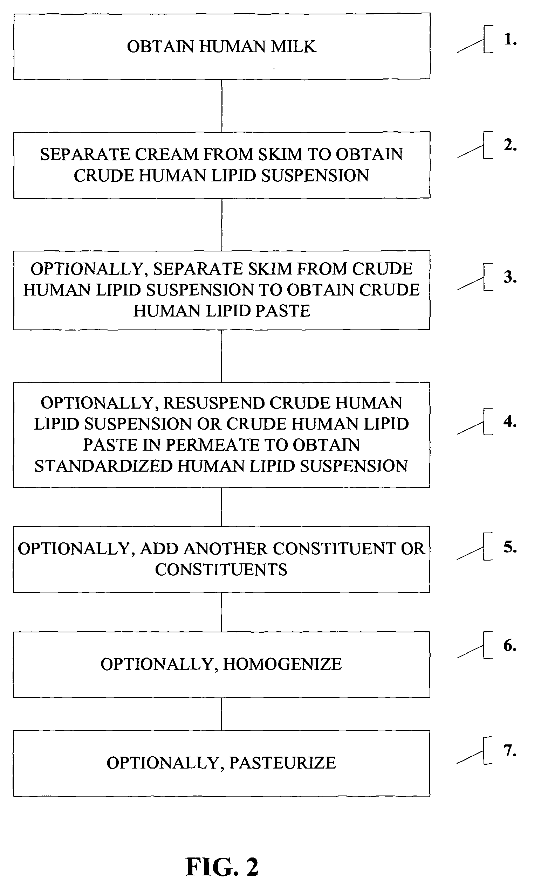

Referring to Fig. 2, step 1, human milk is obtained from donors, as described above. If frozen, the milk can be thawed, pooled, warmed to about 25°C, and genetically screened for contaminants, e.g., viral contaminants, as described in U.S.S.N. 11/947,580. The milk then undergoes filtering, e.g., through about a 200 micron filter, and heat treatment (e.g., at about 63°C or greater for about 30 minutes or more). The milk is transferred to a separator, e.g., a centrifuge, to separate the cream from the skim (step 2). This process intermediate is referred to as Crude Human Lipid Suspension. The skim can be transferred into a second processing tank, e.g., until a filtration step.

In an optional step 3, the cream component can be separated once more (e.g., by centrifugation) to remove additional skim. This process intermediate is referred to as Crude Human Lipid Paste.

In an optional step 4, either the Crude Human Lipid Suspension or the Crude Human Lipid Paste can be re-suspended in excess permeate from the HMF manufacturing process (of U.S.S.N. 11/947,580) to achieve a specific lipid density. This process intermediate is referred to as Standardized Human Lipid Suspension. In the HMF process, following separation of cream and skim the skim undergoes further filtration, e.g., ultrafiltration. This process concentrates the nutrients in the skim milk by filtering out the water. The water obtained during the concentration is referred to as the permeate. This permeate can be used in optional step 4 of the present methods.

In an optional step 5 additional constituent(s) can be added to the resulting composition. The constituents can include: an emulsifier, a preservative, a diluent, an osmolality modifier, and a nutritional component (e.g., mineral, vitamin, and carbohydrate). Examples of such constituents are discussed below.

In an optional step 6, the Standardized Human Lipid Suspension is homogenized, using any method familiar to one skilled in the art. Homogenization removes phospholipids from the membranes. The homogenization step can be carried out earlier than step 6 of the process.

In an optional step 7, the Standardized Human Lipid Suspension (homogenized or non-homogenized) can be pasteurized prior to filling into a suitable container (e.g., a bottle or an oral syringe). Pasteurization methods are known in the art, for example the Suspension can be pasteurized, e.g., at a minimum of about 66 degrees Celsius with the air space maintained at about 69 degrees Celsius for a minimum of about thirty minutes. In one embodiment, the pasteurization can be a short-time (less than about 10 minutes) and ultra-high temperature pasteurization.

Specific order and/or combination of the steps outlined above can be adjusted, if desired.

### Human Lipid Compositions for TPN and Enteral Administration

The present disclosure features compositions of human lipids useful, e.g., in TPN and enteral administration. The compositions can be obtained by the methods discussed herein.

In one embodiment, the composition includes a human lipid fraction. The lipid fraction can be pasteurized and/or can include polyunsaturated fatty acids, e.g., omega-3 and omega-6 fatty acids (or their derivatives or precursors). The composition can be administered parenterally or enterally. The lipid fraction of the administered composition can provide a source of energy to the subject.

In another embodiment, the composition can include a human lipid and an emulsifier. Emulsifiers can include, e.g., egg yolk phospholipids, hydrogenated egg yolk phospholipids, soybean phospholipids, hydrogenated soybean phospholipids or nonionic surfactants. Emulsifiers can also be, e.g., a purified egg yolk lecithin, a purified soybean lecithin and hydrogenated derivatives thereof, nonionic surfactants, such as Polysorbate 80 and HCO-60. One or more of emulsifiers can also be used in combination in the present enteral and parenteral compositions.

In one embodiment, the compositions described herein can include: about 1-20% (w/v of total emulsion composition) of an emulsifier; about 0.5-50% (w/v) of oil, e.g., 5-30% (w/v); about 0.1-80% (w/v) of phospholipids, e.g., 0.1-20% (w/v); and about 0.5-5% of omega-3 fatty acids or derivatives thereof.

The compositions can also include an osmolality modifier, e.g., a polyhydric alcohol, for regulating viscosity of the compositions. The polyhydric alcohols can include, e.g., glycerol and a polyhydric sugar alcohol (e.g., xylitol, sorbitol, and mannitol). Other osmolality modifiers can include glycerin, alanine, sterile water and other modifiers known in the art. One or more osmolality modifiers can also be used in combination in the present compositions.

Optionally, enteral and parenteral compositions described herein can include other constituents, e.g., monoglycerides of fatty acids, diluents (e.g., sugars, starches, lactose, sucrose), preservatives (e.g., antioxidants and anti-microbials), components for adjusting stability (e.g., amino acids), carbohydrates (e.g., fructose and glucose), vitamins, and minerals.

Antioxidants can be added to the compositions to, e.g., protect the unsaturated omega-3 and omega-6 fatty acids (and their precursors and derivatives) from oxidation. Such antioxidants can include alpha-tocopherol (Vitamin E), Vitamin C, carotenoids or retinoids. Other antioxidants that protect the unsaturated omega-3 fatty acids from oxidation after administration and incorporation into biological membranes can also be used.

The emulsion compositions featured herein can be prepared by ways known in the art. For example, the lipids can be mixed with the aqueous phase, the phospholipids (and optionally other emulsifiers), and auxiliary agents in a suitable mixing device. The blend is then homogenized to a desired particle size.

The compositions can also contain stabilizers, such as A-carrageenan. A-carrageenan increases the viscosity of a formula without forming a gel structure, thus retarding the precipitation of insoluble calcium and phosphorus salts if included in the formula. Xanthan gum or other standard stabilizers may also be used.

Flavoring may also be added to the emulsion to make it more palatable for enteral use. Flavoring can be in a form of flavored extracts, volatile oils, chocolate flavoring, peanut butter flavoring, cookie crumbs, vanilla or any commercially available flavoring.

### Use of the Compositions

The compositions featured herein can be administered to subjects, e.g., human subjects, in need of nutritional supplementation, e.g., critically-ill patients, post-operative patients, patients suffering from a nutritional disease or disorder, and/or premature infants.

The embodiments to be considered in all respects as illustrative and not restrictive.

### Example 1. Obtaining Human Lipid Composition for TPN

The following method will be used to obtain a human lipid composition for TPN administration. This method will utilize some components generated in the methods of obtaining human milk fortifiers (HMFs) described in U.S. Application Serial No. 11/947,580 filed on November 29, 2007, as further explained below.
1. Whole milk will be thawed (if frozen), pooled, and genetically screened for any contaminants, e.g., viral contaminants, as described in U.S. Application Serial No. 11/947,580.
2. Whole milk will be warmed to approximately 25°C and separated in to skim milk and cream components (e.g., by centrifugation). For example, the separation will occur as described for HMF manufacturing in U.S.S.N. 11/947,580. The whole milk will be warmed to 25°C, filtered (e.g., through about a 200 micron filter), heat-treated at about 63°C or greater for about 30 minutes, and transferred to a separator, e.g., a centrifuge. After separation in to cream and skim, the cream process intermediate is referred to as Crude Human Lipid Suspension A.
3. Optionally, the cream component will be separated once more (e.g., by centrifugation) to remove additional skim. This process intermediate is referred to as Crude Human Lipid Paste.
4. Optionally, the Human Lipid Paste will be re-suspended in excess permeate from the HMF manufacturing process of U.S.S.N. 11/947,580. In the HMF manufacturing process, the skim portion separated from the cream undergoes further concentration, e.g., ultrafiltration, to filter out water. The filtered out water is referred to as permeate. This permeate can be used in the method described herein. This process intermediate is referred to as Crude Human Lipid Suspension A1.
5. Any remaining protein components hot bound to lipid will be precipitated from solution by manipulating various parameters (e.g., temperature, ionic strength, and solvent (e.g., ethanol or PEG concentration)). These techniques are known to those skilled in the art.
6. Precipitated proteins will be separated from the lipid suspension (e.g., by centrifugation). This process intermediate is referred to as Human Lipid Suspension B.
7. Optionally, soluble and insoluble salts, other ions, and small molecular entities will be removed from the lipid suspension via a purification process (e.g., diafiltration). This process intermediate is referred to as Diafiltrate A.
8. The diafiltrate will be dewatered (e.g., by ultrafiltration). This process intermediate is referred to as Purified Human Lipid Suspension. If desired, emulsifiers and/or preservatives will be added.
9. The Purified Human Lipid Suspension will be pasteurized, yielding a final product.

### Example 2. Obtaining Human Lipid Composition for Enteral Nutrition

The following method will be used to obtain a human lipid composition for enteral administration. This method will utilize some components generated in the methods of obtaining human milk fortifiers (HMFs) described in U.S. Application Serial No. 11/947,580 filed on November 29, 2007, as further explained below.
1. Whole milk will be thawed (if frozen), pooled, and genetically screened for any contaminants, e.g., viral contaminants, as described in U.S. Application Serial No. 11/947,580.
2. Whole milk will be warmed to approximately 25°C and separated in to skim milk and cream components (e.g., by centrifugation). For example, the separation will occur as described for HMF manufacturing in U.S.S.N. 11/947,580. The whole milk will be warmed to 25°C, filtered (e.g., through about a 200 micron filter), heat-treated at about 63°C or greater for about 30 minutes, and transferred to a separator, e.g., a centrifuge. After separation in to cream and skim, the cream process intermediate is referred to as Crude Human Lipid Suspension.
3. Optionally, the cream component may be separated once more (e.g. by centrifugation) to remove additional skim. This process intermediate is referred to as Crude Human Lipid Paste.
4. Optionally, either the Crude Human Lipid Suspension or the Crude Human Lipid Paste will be re-suspended in excess permeate from the HMF manufacturing process (of U.S.S.N. 11/947,580) to achieve specific lipid density. In the HMF manufacturing process, the skim portion separated from the cream undergoes further concentration, e.g., ultrafiltration, to filter out water. The filtered out water is referred to as permeate. This permeate can be used in the method described herein. This process intermediate is referred to as Standardized Human Lipid Suspension.
5. Optionally, the Standardized Human Lipid Suspension will be homogenized.
6. Optionally, the standardized suspension will be pasteurized prior to filling into a suitable container (e.g., a bottle or an oral syringe).

## Claims

1. A method of making a composition that includes a human lipid, the method comprising:
(a) obtaining whole human milk;
(b) separating the milk into a cream portion and a skim portion;
(c) processing the cream portion obtained in step (b) wherein the processing the cream portion comprises purifying the cream portion;
(d) pasteurizing the processed cream portion, thereby obtaining a composition comprising a human lipid, and
(e) preparing the composition obtained in step (d) as a formula for parenteral administration to a human subject.

2. The method of claim 1 further comprising:
filtering the water out of the skim portion after step (b) and
suspending the cream portion obtained in step (b) with the water obtained from filtering the skim portion.

3. The method of claim 1 or 2 wherein processing the cream portion includes purifying the cream portion with diafiltration.

4. The method of one of the preceding claims further comprising ultrafiltering.

5. The method of any one of the preceding claims further comprising adding one or more constituents to the composition obtained in step (c) selected from the group consisting of: an emulsifier, a preservative, a diluent, and an osmolality modifier.

6. The method of claim 5 wherein the preservative is an antioxidant.

7. The method of claim 6 wherein the antioxidant is selected from the group consisting of alpha-tocopheral, vitamin C, a carotenoid, and a retinoid.

8. The method of claim 5 wherein the osmolality modifier comprises glycerin.

9. The method of any one of the preceding claims further comprising adding one or more constituents to the composition obtained in step (c) selected from the group consisting of: a vitamin, a mineral and a carbohydrate.

10. Use of a composition comprising a human lipid in a pasteurized cream portion of whole human milk in the manufacture of a medicament for parenteral administration to a human subject.

11. Use according to claim 10 wherein the manufacture comprises a method according to any one of claims 1 to 9.

12. A parenteral composition comprising a human lipid in a pasteurized cream portion of whole human milk for use in a method of treatment of a human subject, the method comprising parenteral administration of the composition to the subject.

13. The method, use or composition for use according to any one of the preceding claims, wherein the subject is a premature infant.

14. The method, use or composition for use according to any one of the preceding claims, wherein the subject has a nutritional disease or disorder.

## Patentansprüche

1. Verfahren zur Herstellung einer Zusammensetzung, die ein menschliches Lipid umfasst, wobei das Verfahren Folgendes umfasst:
(a) das Gewinnen von menschlicher Vollmuttermilch;
(b) das Trennen der Milch in einen Sahneteil und einen Magermilchteil;
(c) das Verarbeiten des in Schritt (b) gewonnenen Sahneteils, worin das Verarbeiten des Sahneteils das Reinigen des Sahneteils umfasst;
(d) das Pasteurisieren des verarbeiteten Sahneteils, wodurch eine Zusammensetzung gewonnen wird, die ein menschliches Lipid umfasst, und
(e) das Herstellen der in Schritt (d) gewonnenen Zusammensetzung als Formel zur parenteralen Verabreichung an ein menschliches Individuum.

2. Verfahren nach Anspruch 1, das des Weiteren Folgendes umfasst:
das Abfiltrieren des Wassers aus dem Magermilchteil nach Schritt (b) und
das Suspendieren des in Schritt (b) gewonnenen Sahneteils mit dem aus dem Filtrieren des Magermilchteils gewonnenen Wasser.

3. Verfahren nach Anspruch 1 oder 2, worin das Verarbeiten des Sahneteils das Reinigen des Sahneteils mittels Diafiltration umfasst.

4. Verfahren nach einem der vorangegangenen Ansprüche, das des Weiteren Ultrafiltration umfasst.

5. Verfahren nach einem der vorangegangenen Ansprüche, das des Weiteren das Zusetzen eines oder mehrerer aus einem Emulgator, einem Konservierungsmittel, einem Verdünnungsmittel und einem Osmolaritätsmodifikator bestehenden Gruppe ausgewählter Bestandteile zu der in Schritt (c) gewonnenen Zusammensetzung umfasst.

6. Verfahren nach Anspruch 5, worin das Konservierungsmittel ein Antioxidans ist.

7. Verfahren nach Anspruch 6, worin das Antioxidans aus der aus α-Tocopheral, Vitamin C, einem Carotinoid und einem Retinoid bestehenden Gruppe ausgewählt ist.

8. Verfahren nach Anspruch 5, worin der Osmolaritätsmodifikator Glycerin umfasst.

9. Verfahren nach einem der vorangegangenen Ansprüche, das des Weiteren das Zusetzen eines oder mehrerer aus der aus einem Vitamin, einem Mineral und einem Kohlenhydrat bestehenden Gruppe ausgewählter Bestandteile zur der in Schritt (c) gewonnenen Zusammensetzung umfasst.

10. Verwendung einer Zusammensetzung, die ein menschliches Lipid in einem pasteurisierten Sahneteil von menschlicher Vollmuttermilch umfasst, zur Herstellung eines Medikaments zur parenteralen Verabreichung an ein menschliches Individuum.

11. Verwendung nach Anspruch 10, worin die Herstellung ein Verfahren nach einem der Ansprüche 1 bis 9 umfasst.

12. Parenterale Zusammensetzung, die ein menschliches Lipid in einem pasteurisierten Sahneteil von menschlicher Vollmuttermilch umfasst, zur Verwendung in einem Verfahren zur Behandlung eines menschlichen Individuums, wobei das Verfahren die parenterale Verabreichung der Zusammensetzung an das Individuum umfasst.

13. Verfahren, Verwendung oder Zusammensetzung zur Verwendung nach einem der vorangegangenen Ansprüche, worin das Individuum ein frühgeborener Säugling ist.

14. Verfahren, Verwendung oder Zusammensetzung zur Verwendung nach einem der vorangegangenen Ansprüche, worin das Individuum eine Ernährungskrankheit oder -störung aufweist.

## Revendications

1. Procédé de fabrication d'une composition qui comprend un lipide humain, le procédé comprenant les étapes consistant à :
(a) obtenir du lait humain entier ;
(b) séparer le lait en une partie de crème et une partie écrémée ;
(c) traiter la partie de crème obtenue à l'étape (b), le traitement de la partie de crème comprenant la purification de la partie de la crème ;
(d) pasteuriser la partie de crème traitée, en obtenant ainsi une composition comprenant un lipide humain, et
(e) préparer la composition obtenue à l'étape (d) sous la forme d'une formule destinée à une administration par voie parentérale à un sujet humain.

2. Procédé selon la revendication 1, comprenant en outre les étapes consistant à :
filtrer l'eau en dehors de la partie écrémée après l'étape (b), et
mettre en suspension la partie de crème obtenue à l'étape (b) avec l'eau provenant de la filtration de la partie écrémée.

3. Procédé selon la revendication 1 ou 2, dans lequel le traitement de la partie de crème comprend la purification de la partie de crème via diafiltration.

4. Procédé selon l'une quelconque des revendications précédentes, comprenant en outre une ultrafiltration.

5. Procédé selon l'une quelconque des revendications précédentes, comprenant en outre l'ajout d'un ou de plusieurs constituants de la composition obtenue à l'étape (c), choisis dans le groupe comprenant : un émulsifiant, un conservateur, un diluant et un agent de modification d'osmolalité.

6. Procédé selon la revendication 5, dans laquelle le conservateur est un antioxydant.

7. Procédé selon la revendication 6, dans lequel l'antioxydant est choisi dans le groupe comprenant l'alpha-Tocophéral, la vitamine C, un caroténoïde et un rétinoïde.

8. Procédé selon la revendication 5, dans lequel le modificateur d'osmolalité comprend de la glycérine.

9. Procédé selon l'une quelconque des revendications précédentes, comprenant en outre l'ajout d'un ou de plusieurs constituants de la composition obtenue à l'étape (c), choisis dans le groupe comprenant : une vitamine, un minéral et un carbohydrate.

10. Utilisation d'une composition comprenant un lipide humain dans une partie de crème pasteurisée de lait humain entier dans la fabrication d'un médicament destiné à une administration parentérale à un sujet humain.

11. Utilisation selon la revendication 10, dans laquelle la fabrication comprend un procédé selon l'une quelconque des revendications 1 à 9.

12. Composition parentérale comprenant un lipide humain dans une partie de crème pasteurisée de lait humain entier, destinée à être utilisée dans un procédé de traitement d'un sujet humain, le procédé comprenant l'administration parentérale de la composition au sujet.

13. Procédé, utilisation ou composition pour une utilisation selon l'une quelconque des revendications précédentes, où le sujet est un enfant prématuré.

14. Procédé, utilisation ou composition pour une utilisation selon l'une quelconque des revendications précédentes, où le sujet souffre d'une maladie ou d'un trouble nutritionnel.
